# EUROPÄISCHE PATENTSCHRIFT

(11) **EP 3 970 648 B1**
(45) Veröffentlichungstag und Bekanntmachung des Hinweises auf die Patenterteilung: **26.04.2023**
(21) Anmeldenummer: 21196238.6
(22) Anmeldetag: 13.09.2021
(51) Int. Cl.: A61B 18/14, A61B 18/00

(54) **MEDIZINISCHES HF-INSTRUMENT**
MEDICAL HIGH FREQUENCY INSTRUMENT
INSTRUMENT MÉDICAL HAUTE FRÉQUENCE

(30) Priorität: 18.09.2020 DE 102020124427
(43) Veröffentlichungstag der Anmeldung: 23.03.2022
(73) Patentinhaber: Karl Storz SE & Co. KG, 78532 Tuttlingen (DE)
(72) Erfinder: Hahn, Martin, 78532 Tuttlingen (DE); Hermle, Rainer, 78532 Tuttlingen (DE); Wittke, Uwe, 78532 Tuttlingen (DE)

(56) Entgegenhaltungen:
- DE-A1-102018 121 733
- DE-B4-102010 035 319
- US-A1- 2019 350 617

## Beschreibung

Die Erfindung betrifft ein medizinisches HF-Instrument mit einem ein hohles Schaftrohr aufweisenden Schaft und einer am proximalen Ende des Schaftrohres angeordneten Handhabe, wobei im Schaftrohr eine HF-Elektrode gelagert ist, die zur Energieversorgung mit einem an der Handhabe festlegbaren HF-Stecker koppelbar ist, wobei die HF-Elektrode über einen federbelasteten Rastmechanismus am HF-Stecker festlegbar ist.

Medizinische HF-Instrumente werden verwendet, um mittels einer mit hochfrequentem Wechselstrom beaufschlagten Schneidvorrichtung schädliches oder beschädigtes Gewebe bei einer endoskopischen Operation abzutragen. Die Energieversorgung der HF-Elektrode erfolgt über einen an der Handhabe festlegbaren HF-Stecker. Die Kopplung des HF-Steckers mit der Handhabe erfolgt in der Regel über eine Rastmechanik. Diese bekannten Rastmechaniken weisen den Nachteil auf, dass diese mit fortschreitender Lebensdauer störungsanfällig werden und sich von der Handhabe lösen können, wodurch keine sichere Kontaktierung mit der HF-Elektrode mehr gewährleistet ist.

Aus der CN 1 01 461 736 A ist ein als bipolarer Elektrokoagulations-Aspirator ausgebildetes HF-Instrument bekannt. Das Kontaktieren des HF-Steckers mit den beiden Polen der HF-Elektrode erfolgt bei diesem bekannten HF-Instrument dadurch, dass ein HF-Stecker in die Handhabe eingesteckt wird, bis eine Elektrode des HF-Steckers in Kontakt mit den Polen der HF-Elektrode tritt.

Weiterhin ist aus der DE 10 2018 121 733 A1 ist eine Kanüle zur Elektrostimulation bekannt, an der zur Stromversorgung ein im Querschnitt U-förmig ausgebildeter Stecker über einen Rastmechanismus festlegbar ist. Die elektrische Kontaktierung des Steckers mit dem Kanülenrohr erfolgt über einen auf der Unterseite des Steckers angeordneten Kontakt, dessen freie Enden als Federzungen ausgebildet sind, die beim Aufsetzen des Steckers nach oben gebogen, werden.

Die US 2019/0350617 A1 offenbart eine Unipolar-Kanüle zur Erzeugung eines elektrischen Stimulationsreizes an einem Nerv. Die Stromversorgung zur Kanüle erfolgt über einen Stecker, der an einem proximalseitigen Ansatz der Kanüle festlegbar ist. Die elektrische Kontaktierung des Steckers mit dem Kanülenrohr erfolgt über einen am Stecker angeordneten Kontakt, der als Federzunge oder Schneid-Klemm-Kontakt ausgebildet ist und beim Aufsetzen des Steckers auf den proximalseitigen Ansatz die elektrische Verbindung zum Kanülenrohr bewirkt.

Aus der DE 10 2010 035 319 B4 ist weiterhin ein Resektoskop mit einer hochfrequenzbeaufschlagbaren Elektrodenanordnung bekannt. Die Verbindung des Resektopskops mit einem HF-Generator erfolgt über einen als Schlitten ausgebildeten Stecker, in den das proximale Ende der mit dem HF-Werkzeug des Resektoskops verbundenen HF-Elektrode einsteckbar ist. Im Inneren des Schlittens erfolgt die Kontaktierung der HF-Elektrode mit dem HF-Generator über elastische Federzungen, die beim Einschieben der HF-Elektrode in den Schlitten mit der HF-Elektrode in Kontakt treten.

Davon ausgehend liegt der Erfindung die Aufgabe zugrunde, ein medizinisches HF-Instrument so auszugestalten, dass der HF-Stecker bei einfachem Aufbau eine zuverlässige Kontaktierung mit der HF-Elektrode gewährleistet. Die Lösung dieser Aufgabenstellung ist erfindungsgemäß dadurch gekennzeichnet, dass der federbelastete Rastmechanismus mindestens ein am HF-Stecker gelagertes Federelement aufweist, wobei der HF-Stecker gegen die Federkraft dieses mindestens einen Federelements in ein Gehäuse der Handhabe eindrückbar ist und wobei der HF-Stecker im Querschnitt U-förmig ausgebildet ist, wobei das mindestens eine Federelement auf einer der Handhabe zugewandten Unterseite mindestens eines der parallelen Schenkel des U-förmigen HF-Steckers angeordnet ist und in mindestens einem der parallelen Schenkel des U-förmigen HF-Steckers eine Aufnahme für die HF-Elektrode ausgebildet ist.

Durch die Ausbildung des Rastmechanismus wird die HF-Elektrode am HF-Stecker festgelegt. Sobald der Rastmechanismus greift, ist auch eine zuverlässige Kontaktierung mit der HF-Elektrode gewährleistet.

Das federbelastete Eindrücken des HF-Steckers in das Gehäuse der Handhabe ist vom Bediener schnell und problemlos auch im Einhandbetrieb durchführbar.

Aufgrund der U-förmigen Ausgestaltung lässt sich der HF-Stecker einfach und lagesicher in einer entsprechenden Steckeraufnahme im Gehäuse der Handhabe so platzieren, dass ein in der Steckeraufnahme ausgebildeter Steg zwischen den beiden parallelen Schenkeln des U-förmigen HF-Steckers Aufnahme findet.

Um sicherzustellen, dass die HF-Elektrode und der HF-Stecker kontaktsicher miteinander gekoppelt sind, wird mit der Erfindung vorgeschlagen, dass die HF-Elektrode nur bei in das Gehäuse der Handhabe eingedrücktem HF-Stecker mit dem HF-Stecker koppelbar ist. Erst das aktive Eindrücken des HF-Steckers in das Gehäuse der Handhabe durch den Bediener des medizinischen HF-Instruments ermöglicht somit die kontaktschließende Kopplung der Bauteile HF-Elektrode und HF-Stecker.

Weiterhin wird mit der Erfindung vorgeschlagen, dass am mit dem HF-Stecker koppelbaren Teil der HF-Elektrode eine Rastfläche ausgebildet ist, die im mit dem HF-Stecker gekoppelten Zustand mit einer korrespondierenden Anlagefläche im Gehäuse der Handhabe zusammenwirkt, um die HF-Elektrode kontaktsicher im Gehäuse der Handhabe und somit auch im HF-Stecker zu halten.

Mit einer praktischen Ausführungsform der Erfindung wird weiterhin vorgeschlagen, dass in beiden parallelen Schenkeln des U-förmigen HF-Steckers jeweils eine Aufnahme für die HF-Elektrode ausgebildet ist, um einen stets sicheren elektrischen Kontakt der HF-Elektrode zu gewährleisten.

Um das Einführen der HF-Elektrode in die Aufnahme zu erleichtern und das Eindringen von Feuchtigkeit in die Aufnahme zu verhindern, wird erfindungegemäß vorgeschlagen, dass die mindestens eine Aufnahme für die HF-Elektrode distalseitig eine Anlaufschräge sowie eine Dichtungslamelle aufweist.

Schließlich wird mit der Erfindung vorgeschlagen, dass auf der Unterseite beider paralleler Schenkel des U-förmigen HF-Steckers jeweils mindestens zwei hintereinander angeordnete Federelemente angeordnet sind.

Weitere Merkmale und Vorteile der Erfindung ergeben sich anhand der zugehörigen Zeichnungen, in denen ein Ausführungsbeispiel eines erfindungsgemäßen medizinischen HF-Instruments nur beispielhaft dargestellt ist, ohne die Erfindung auf dieses Ausführungsbeispiel zu beschränken. In den Zeichnungen zeigt:
- Fig. 1: eine perspektivische Seitenansicht eines erfindungsgemäßen medizinischen HF-Instruments;
- Fig. 2: eine vergrößerte perspektivische Ansicht von unten einer ersten Ausführungsform des HF-Steckers gemäß Fig. 1 ohne das medizinische HF-Instrument;
- Fig. 3: einen vergrößerten ausschnittweisen Längsschnitt durch das Detail III gemäß Fig.1, den HF-Stecker in einer Position vor dem Koppeln mit der HF-Elektrode darstellend;
- Fig. 4: einen Längsschnitt gemäß Fig. 3, jedoch den HF-Stecker in der mit der HF-Elektrode koppelbaren Position darstellend;
- Fig. 5: einen Längsschnitt gemäß Fig. 3, jedoch den HF-Stecker in der mit der HF-Elektrode gekoppelten Position darstellend;
- Fig. 6: eine vergrößerte perspektivische Ansicht von unten einer zweiten Ausführungsform des HF-Steckers gemäß Fig. 1, die nicht Gegenstand der Erfindung ist, ohne das medizinische HF-Instrument und
- Fig. 7: einen vergrößerten ausschnittweisen Längsschnitt durch das Detail VII, das nicht Gegenstand der Erfindung ist, gemäß Fig.1, den HF-Stecker in der mit der HF-Elektrode gekoppelten Position darstellend.

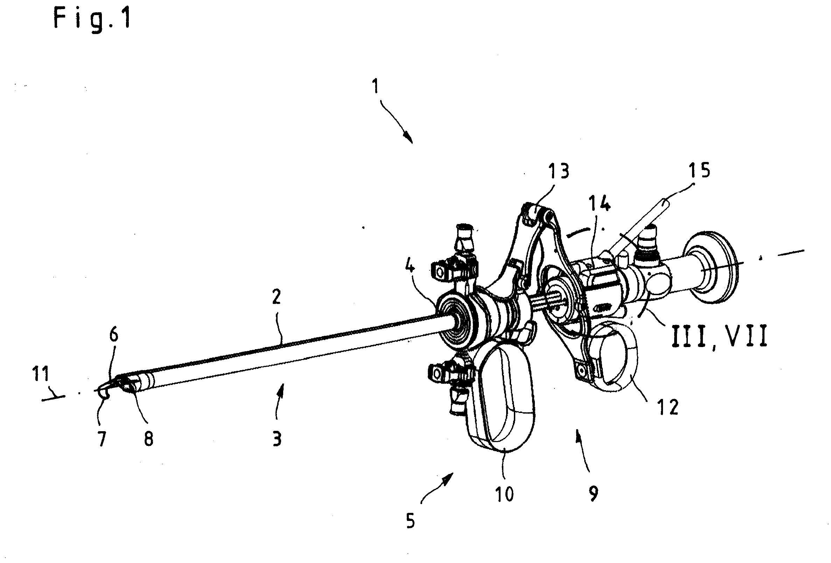

Die Abbildung Fig. 1 zeigt ein medizinisches HF-Instrument 1 mit einem ein hohles Schaftrohr 2 aufweisenden Schaft 3, wobei am proximalen Ende 4 des Schaftrohres 2 eine Handhabe 5 angeordnet ist.

Bei dem beispielhaft dargestellten medizinischen HF-Instrument 1 handelt es sich um ein Resektoskop mit einer im Schaftrohr 2 des Schaftes 3 gelagerten Hochfrequenz-Elektrode 6 (HF-Elektrode), die distalseitig als HF-Schlinge 7 ausgebildet ist und die zu Operationszwecken aus dem distalen Ende 8 des Schaftrohres 2 herausfahrbar ist. Zum Einziehen der HF-Elektrode 6 in das Schaftrohr 2 bzw. zum Herausfahren der HF-Elektrode 6 aus dem Schaftrohr 2 weist die Handhabe 5 als Antrieb 9 einen starren, fest mit dem Schaftrohr 2 verbundenen distalen Teil 10 sowie einen in Richtung der Längsachse 11 des Schaftes 3 verschiebbaren proximalen Teil 12 auf, wobei der starre distale Teil 10 und der verschiebbare proximale Teil 12 über einen Gelenkmechanismus 13 miteinander verbunden sind.

Alternativ zu der in Fig. 1 dargestellten Ausführungsform zur Ausbildung des Antriebs 9 ist es selbstverständlich auch möglich, den proximalen Teil 12 starr und den distalen Teil 10 in Richtung der Längsachse 11 des Schaftes 3 verschiebbar auszubilden.

Zur Energieversorgung der HF-Elektrode 6 ist die HF-Elektrode 6 mit einem an der Handhabe 5 festlegbaren HF-Stecker 14 koppelbar, der über ein Kabel 15 mit einer externen Energiequelle verbunden ist

Bei der in Fig. 1 dargestellten Ausführungsform des medizinischen HF-Instruments 1 ist der HF-Stecker 14 am verschiebbaren proximalen Teil 12 der Handhabe 5 angeordnet. Die Lage des HF-Steckers 14 an der Handhabe 5 ist jedoch ebenso wie die Art der Ausgestaltung der Handhabe 5 für die Funktion des HF-Steckers 14 unerheblich.

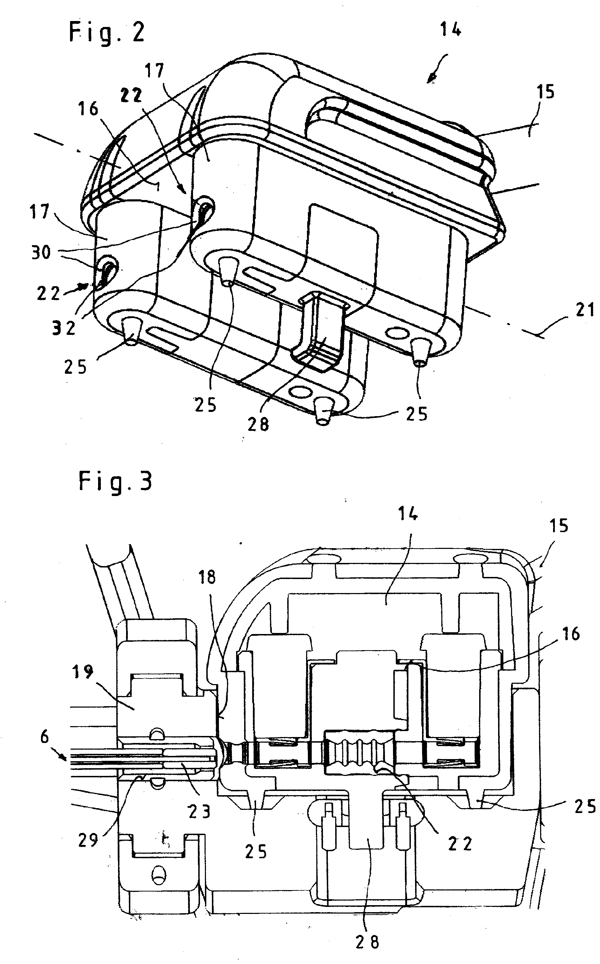

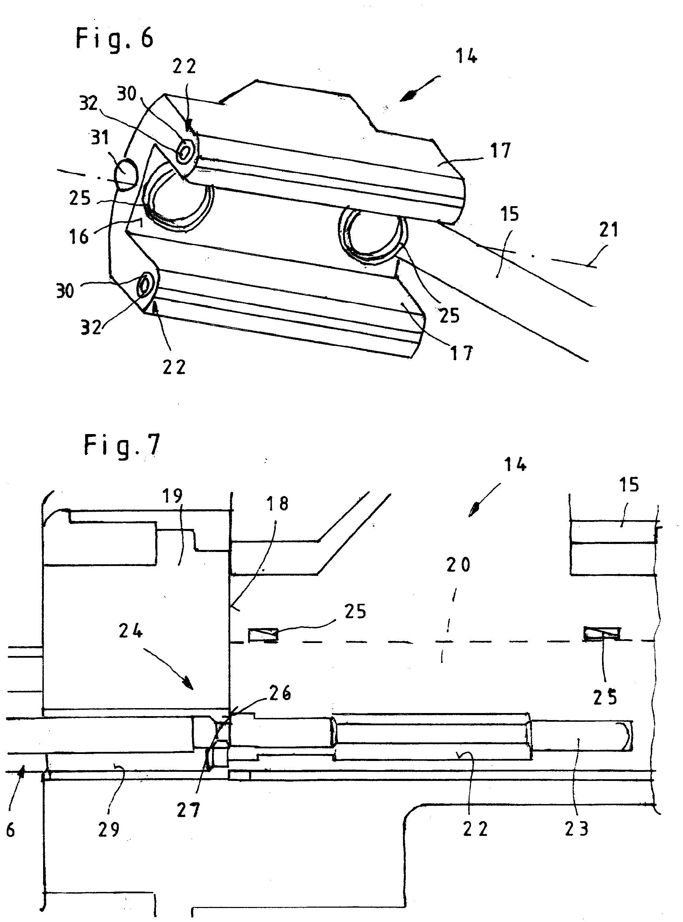

In den Abbildungen Fig. 2 und 6 sind zwei unterschiedliche Ausführungsformen zur Ausbildung des HF-Steckers 14 dargestellt.

Wie aus Fig. 2 und 6 ersichtlich, weist jeder der im Querschnitt U-förmig ausgebildeten HF-Stecker 14 eine horizontale Grundfläche 16 und zwei parallel und mit Abstand zueinander angeordnete und senkrecht auf der Grundfläche 16 aufstehende Schenkel 17 auf. Mit den beiden parallelen Schenkeln 17 voran ist der jeweilige HF-Stecker 14 in eine Steckeraufnahme 18 im Gehäuse 19 der Handhabe 5 so einsetzbar, dass ein in der Steckeraufnahme 18 ausgebildeter Steg 20 zwischen den beiden parallelen Schenkeln 17 des U-förmigen HF-Steckers 14 Aufnahme findet.

Durch das sattelförmige Aufsetzen des HF-Steckers 14 auf den Steg 20 im Gehäuse 19 der Handhabe 5 ist der HF-Stecker 14 einfach und schnell lagesicher mit der Handhabe 5 verbindbar.

Zum Kontaktieren des HF-Steckers 14 mit der HF-Elektrode 6 ist in den beiden parallelen Schenkeln 17 des U-förmigen HF-Steckers 14 jeweils eine sich in Richtung der Längsachse 21 des HF-Steckers 14 erstreckende Aufnahme 22 für die HF-Elektrode 6 ausgebildet, wozu am proximalen Ende der HF-Elektrode 6 zwei parallel und mit Abstand zueinander angeordnete Kontaktstifte 23 ausgebildet sind, die in die Aufnahmen 22 einführbar sind.

Um einerseits eine sichere Kontaktierung zwischen der HF-Elektrode 6 und dem HF-Stecker 14 zu gewährleisten und andererseits ein lagefeste Arretierung des HF-Steckers 14 und der HF-Elektrode 6 in der Handhabe 5 zu ermöglichen, ist ein federbelasteter Rastmechanismus 24 vorgesehen.

Der federbelastete Rastmechanismus 24 besteht aus mindestens einem am HF-Stecker 14 angeordneten Federelement 25, gegen dessen Rückstellkraft der HF-Stecker 14 in das Gehäuse 19 der Handhabe 5 eindrückbar ist, sowie aus einer an der HF-Elektrode 6 ausgebildeten Rastfläche 26 und einer korrespondierenden, im Gehäuse 19 der Handhabe 5 ausgebildeten Anlagefläche 27.

Bei der in Fig. 2 bis 5 dargestellten ersten Ausführungsform des HF-Steckers 14 sind zur Ausbildung des Rastmechanismus 24 auf den Unterseiten der parallelen Schenkel 17 des U-förmigen HF-Steckers 14 in Richtung der Längsachse 21 des HF-Steckers 14 hintereinander jeweils zwei Federelemente 25 aus einem elastisch verformbaren Kunststoff-Material, vorzugsweise einem Elastomer, angeordnet.

Das Einsetzen dieses HF-Steckers 14 in das Gehäuse 19 der Handhabe 5 sowie das Koppeln der HF-Elektrode 6 mit dem HF-Stecker 14 wird nachfolgend anhand der Abbildungen Fig. 3 bis 5 beschrieben.

Zum Verbinden des HF-Steckers 14 mit der Handhabe 5 wird der U-förmig ausgebildete HF-Stecker 14 mit den beiden parallelen Schenkeln 17 voran in die Steckeraufnahme 18 im Gehäuse 19 der Handhabe 5 eingeführt, bis die auf der Unterseite der Schenkel 17 des U-förmigen HF-Steckers 14 angeordneten Federelemente 25 auf dem Grund der Steckeraufnahme 18 aufliegen und der Steg 20 zwischen den beiden parallelen Schenkeln 17 des U-förmigen HF-Steckers 14 Aufnahme findet, wie dies in Fig. 7 dargestellt ist. In dieser Grundstellung des HF-Steckers 14 im Gehäuse 19 der Handhabe 5 ist der HF-Stecker 14 über einen Rasthaken 28 am Grundkörper 16 des U-förmigen HF-Steckers 14 in der Steckeraufnahme 18 gegen Herausfallen aus der Handhabe 6 gesichert.

Um ausgehend von der in Fig. 3 dargestellten Grundstellung des HF-Steckers 14 den HF-Stecker 14 mit der HF-Elektrode 6 zu koppeln, muss auf den HF-Stecker 14 eine senkrechte Druckkraft D ausgeübt werden, wodurch der HF-Stecker 14 gegen die Rückstellkraft der Federelemente 25 tiefer in die Steckeraufnahme 18 im Gehäuse 19 der Handhabe 5 gedrückt wird, bis die in den parallelen Schenkeln 17 ausgebildeten Aufnahmen 22 mit Durchgangsbohrungen 29 im Gehäuse 19 der Handhabe 5 fluchten, in denen die das proximale Ende der HF-Elektrode 6 bildenden Kontaktstifte 23 gelagert sind.

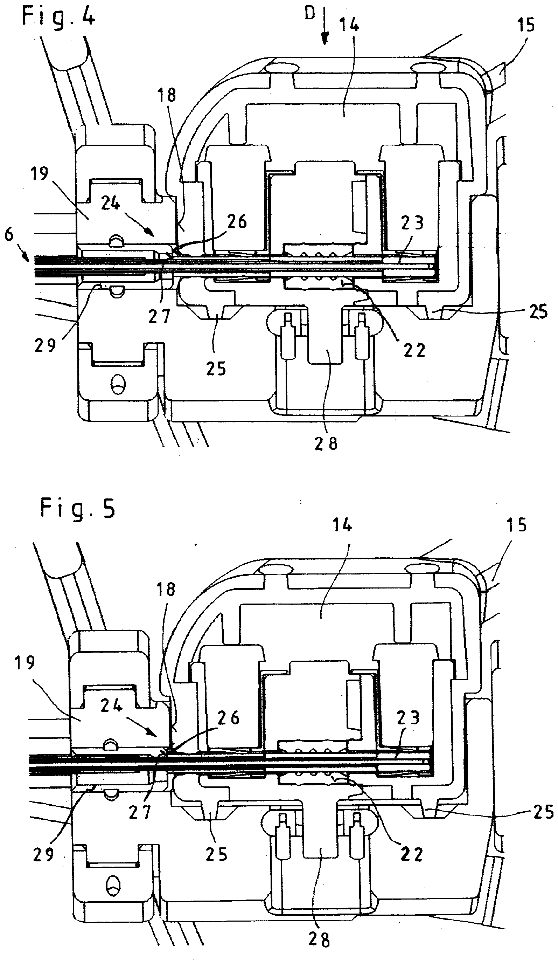

In dieser in die Handhabe 5 eingedrückten, in Fig. 4 dargestellten Position des HF-Steckers 14, können nun die Kontaktstifte 23 der HF-Elektrode 6 nach proximal in die Aufnahmen 22 des HF-Steckers 14 geschoben werden, um die HF-Elektrode 6 mit dem HF-Stecker 14 elektrisch zu kontaktieren. Das federbelastete Eindrücken des HF-Steckers 14 in das Gehäuse 19 der Handhabe 5 ist vom Bediener schnell und problemlos auch im Einhandbetrieb durchführbar.

Alternativ zum Eindrücken des HF-Steckers 14 in die Steckeraufnahme 18 mittels der Druckkraft D kann das Überführen des HF-Steckers 14 in die Kontaktierposition gemäß Fig. 4 auch dadurch bewirkt werden, dass die HF-Elektrode 6 mit den beiden Kontaktstiften 23 voran nach proximal in die Aufnahmen 22 des HF-Steckers 14 geschoben wird. Hierzu weisen die Aufnahmen 22 distalseitig vorteilhafterweise Anlaufschrägen 30 auf, die das Eintreten der Kontaktstifte 23 in die Aufnahmen 22 erleichtern. Durch die in die Aufnahmen 22 eintretenden Kontaktstifte 23 wird der HF-Stecker 14 gegen die Rückstellkraft der Federelemente 25 tiefer in die Steckeraufnahme 18 gedrückt.

Die HF-Elektrode 6 ist ausschließlich in der in das Gehäuse 19 der Handhabe 5 eingedrückten Position des HF-Steckers 14 mit dem HF-Stecker 14 koppelbar und ermöglicht die kontaktschließende Kopplung der Bauteile HF-Elektrode 6 und HF-Stecker 14.

Sobald nun keine Druckkraft D mehr auf den HF-Stecker 14 ausgeübt wird, drücken auf dem Grund der Steckeraufnahme 18 aufliegenden Federelemente 25 den HF-Stecker 14 wieder senkrecht nach oben in Richtung der in Fig. 3 dargestellten Grundstellung.

Diese Aufwärtsbewegung endet, sobald die aus den Durchgangsbohrungen 29 in die Aufnahmen 22 ragenden Kontaktstifte 23 gegen die obere Wandung der Durchgangsbohrungen 29 anlaufen. In dieser in Fig. 5 dargestellten Rastposition, liegen die an den Kontaktstiften 23 ausgebildete Rastflächen 26 an den korrespondierenden Anlageflächen 27 des Gehäuses 19 der Handhabe 5 an und verhindern so ein axiales Herausziehen der Kontaktstifte 23 nach distal aus den Aufnahmen 22 des HF-Steckers 14.

Diese Verrastung der Kontaktstifte 23 der HF-Elektrode 6 mit dem HF-Stecker 14 und dem Gehäuse 19 der Handhabe 5 gewährleistet so eine sichere Kontaktierung zwischen der HF-Elektrode 6 und dem HF-Stecker 14 und darüber hinaus auch einen sicheren Halt des HF-Steckers 14 in der Handhabe 5.

Um die Kontaktierung des HF-Steckers 14 mit der HF-Elektrode 6 wieder zu lösen, muss der HF-Stecker 14 durch erneutes Aufbringen einer Druckkraft D wieder in die Handhabe 5 eingedrückt werden, bis die Rastflächen 26 der Kontaktstifte 23 der HF-Elektrode 6 wieder außer Kontakt mit den Anlageflächen 27 des Gehäuses 19 der Handhabe 5 treten. In dieser in Fig. 4 dargestellten Position lassen sich die Kontaktstifte 23 der HF-Elektrode 6 wieder leicht nach distal aus den Aufnahmen 22 des HF-Steckers 14 ziehen.

Bei der in Fig. 6 und 7 dargestellten zweiten Ausführungsform des HF-Steckers 14, die nicht Gegenstand der Erfindung ist, sind zur Ausbildung des Rastmechanismus 24 auf der den parallelen Schenkeln 17 zugewandten Unterseite der Grundfläche 16 des U-förmigen HF-Steckers 14 und in Richtung der Längsachse 21 des HF-Steckers 14 hintereinander zwei als Schraubenfedern ausgebildete Federelemente 25 angeordnet.

Das Einsetzen dieses HF-Steckers 14 in das Gehäuse 19 der Handhabe 5 sowie das Koppeln der HF-Elektrode 6 mit dem HF-Stecker 14 wird nachfolgend anhand der Abbildung Fig. 7 sowie mit Verweis auf die Abbildungen Fig. 3 und 4 der ersten Ausführungsformbeschrieben.

Zum Verbinden des HF-Steckers 14 mit der Handhabe 5 wird der U-förmig ausgebildete HF-Stecker 14 mit den beiden parallelen Schenkeln 17 voran in die Steckeraufnahme 18 im Gehäuse 19 der Handhabe 5 eingeführt, bis die auf der Unterseite der Grundfläche 16 des U-förmigen HF-Steckers 14 angeordneten Federelemente 25 auf dem in der Steckeraufnahme 18 ausgebildeten und in Richtung der Längsachse 11 des Schaftes 3 ausgerichteten Steg 20 aufliegen und der Steg 20 zwischen den beiden parallelen Schenkeln 17 des U-förmigen HF-Steckers 14 Aufnahme findet. In dieser Grundstellung des HF-Steckers 14 im Gehäuse 19 der Handhabe 5 ist der HF-Stecker 14 über distalseitig und proximalseitig am Grundkörper 16 des U-förmigen HF-Steckers 14 angeordnete Kugelrasten 31 in der Steckeraufnahme 18 gegen Herausfallen aus der Handhabe 6 gesichert. Bis auf die Anordnung der Federelemente 25 am HF-Stecker 14 sowie die Ausbildung der Federelemente 25 an sich, entspricht diese Grundstellung der in Fig. 3 dargestellten Grundstellung der zuvor beschriebenen ersten Ausführungsform des HF-Steckers 14.

Um ausgehend von dieser Grundstellung des HF-Steckers 14 den HF-Stecker 14 mit der HF-Elektrode 6 zu koppeln, muss auf den HF-Stecker 14 eine senkrechte Druckkraft D ausgeübt werden, wodurch der HF-Stecker 14 gegen die Rückstellkraft der Federelemente 25 tiefer in die Steckeraufnahme 18 im Gehäuse 19 der Handhabe 5 gedrückt wird, bis die in den parallelen Schenkeln 17 ausgebildeten Aufnahmen 22 mit Durchgangsbohrungen 29 im Gehäuse 19 der Handhabe 5 fluchten, in denen die das proximale Ende der HF-Elektrode 6 bildenden Kontaktstifte 23 gelagert sind.

In dieser in Fig. 4 zur ersten Ausführungsform des HF-Steckers 14 dargestellten, in die Handhabe 5 eingedrückten Position des HF-Steckers 14 können nun die Kontaktstifte 23 der HF-Elektrode 6 nach proximal in die Aufnahmen 22 des HF-Steckers 14 geschoben werden, um die HF-Elektrode 6 mit dem HF-Stecker 14 elektrisch zu kontaktieren. Das federbelastete Eindrücken des HF-Steckers 14 in das Gehäuse 19 der Handhabe 5 ist vom Bediener schnell und problemlos auch im Einhandbetrieb durchführbar.

Alternativ zum Eindrücken des HF-Steckers 14 in die Steckeraufnahme 18 mittels der Druckkraft D kann das Überführen des HF-Steckers 14 in die Kontaktierposition auch dadurch bewirkt werden, dass die HF-Elektrode 6 mit den beiden Kontaktstiften 23 voran nach proximal in die Aufnahmen 22 des HF-Steckers 14 geschoben wird. Hierzu weisen die Aufnahmen 22 distalseitig vorteilhafterweise Anlaufschrägen 30 auf, die das Eintreten der Kontaktstifte 23 in die Aufnahmen 22 erleichtern. Durch die in die Aufnahmen 22 eintretenden Kontaktstifte 23 wird der HF-Stecker 14 gegen die Rückstellkraft der Federelemente 25 tiefer in die Steckeraufnahme 18 gedrückt.

Die HF-Elektrode 6 ist ausschließlich in der in das Gehäuse 19 der Handhabe 5 eingedrückten Position des HF-Steckers 14 mit dem HF-Stecker 14 koppelbar und ermöglicht die kontaktschließende Kopplung der Bauteile HF-Elektrode 6 und HF-Stecker 14.

Sobald nun keine Druckkraft D mehr auf den HF-Stecker 14 ausgeübt wird, drücken die auf dem Steg 20 aufliegenden Federelemente 25 den HF-Stecker 14 wieder senkrecht nach oben in Richtung der Grundstellung.

Diese Aufwärtsbewegung endet, sobald die aus den Durchgangsbohrungen 29 in die Aufnahmen 22 ragenden Kontaktstifte 23 gegen die obere Wandung der Durchgangsbohrungen 29 anlaufen. In dieser in Fig. 7 dargestellten Rastposition, liegen die an den Kontaktstiften 23 ausgebildete Rastflächen 26 an den korrespondierenden Anlageflächen 27 des Gehäuses 19 der Handhabe 5 an und verhindern so ein axiales Herausziehen der Kontaktstifte 23 nach distal aus den Aufnahmen 22 des HF-Steckers 14.

Diese Verrastung der Kontaktstifte 23 der HF-Elektrode 6 mit dem HF-Stecker 14 und dem Gehäuse 19 der Handhabe 5 gewährleistet so eine sichere Kontaktierung zwischen der HF-Elektrode 6 und dem HF-Stecker 14 und darüber hinaus auch einen sicheren Halt des HF-Steckers 14 in der Handhabe 5.

Um die Kontaktierung des HF-Steckers 14 mit der HF-Elektrode 6 wieder zu lösen, muss der HF-Stecker 14 durch erneutes Aufbringen einer Druckkraft D wieder in die Handhabe 5 eingedrückt werden, bis die Rastflächen 26 der Kontaktstifte 23 der HF-Elektrode 6 wieder außer Kontakt mit den Anlageflächen 27 des Gehäuses 19 der Handhabe 5 treten. In dieser Position lassen sich die Kontaktstifte 23 der HF-Elektrode 6 wieder leicht nach distal aus den Aufnahmen 22 des HF-Steckers 14 ziehen.

Wie weiterhin aus Fig. 2 und 6 ersichtlich, sind im Bereich des distalen Endes der Aufnahmen 22 für die Kontaktstifte 23 der HF-Elektrode 6 Dichtungslamellen 32 angeordnet, die in der mit der HF-Elektrode 6 kontaktierten Position abdichtend an den Kontaktstiften 23 anliegen, um das Eindringen von Feuchtigkeit in die Aufnahmen 22 zu verhindern.

Ein wie zuvor beschreiben aufgebautes medizinisches HF-Instrument 1 zeichnet sich dadurch aus, dass der HF-Stecker 14 bei einfachem Aufbau eine zuverlässige Kontaktierung mit der HF-Elektrode 6 gewährleistet und gleichzeitig schnell und sicher in der Handhabe 5 festlegbar ist.

### Bezugszeichenliste

- 1: medizinisches HF-Instrument
- 2: Schaftrohr
- 3: Schaft
- 4: proximales Ende (Schaftrohr)
- 5: Handhabe
- 6: HF-Elektrode
- 7: HF-Schlinge
- 8: distales Ende (Schaftrohr)
- 9: Antrieb
- 10: starres distales Teil (Handhabe)
- 11: Längsachse (Schaft)
- 12: verschiebbares proximales Teil (Handhabe)
- 13: Gelenkmechanismus
- 14: HF-Stecker
- 15: Kabel
- 16: Grundfläche
- 17: Schenkel
- 18: Steckeraufnahme
- 19: Gehäuse
- 20: Steg
- 21: Längsachse (HF-Stecker)
- 22: Aufnahme
- 23: Kontaktstift
- 24: Rastmechanismus
- 25: Federelement
- 26: Rastfläche
- 27: Anlagefläche
- 28: Rasthaken
- 29: Durchgangsbohrung
- 30: Anlaufschräge
- 31: Kugelraste
- 32: Dichtungslamelle

- D: Druckkraft

## Patentansprüche

1. Medizinisches HF-Instrument mit einem ein hohles Schaftrohr (2) aufweisenden Schaft (3) und einer am proximalen Ende (4) des Schaftrohres (2) angeordneten Handhabe (5), wobei im Schaftrohr (2) eine HF-Elektrode (6) gelagert ist, die zur Energieversorgung mit einem an der Handhabe (5) festlegbaren HF-Stecker (14) koppelbar ist, wobei die HF-Elektrode (6) über einen federbelasteten Rastmechanismus (24) am HF-Stecker (14) festlegbar ist, wobei der federbelastete Rastmechanismus (24) mindestens ein am HF-Stecker (14) gelagertes Federelement (25) aufweist, wobei der HF-Stecker (14) gegen die Federkraft dieses mindestens einen Federelements (25) in ein Gehäuse (19) der Handhabe (5) eindrückbar ist und wobei der HF-Stecker (14) im Querschnitt U-förmig ausgebildet ist, wobei das mindestens eine Federelement (25) auf einer der Handhabe (5) zugewandten Unterseite mindestens eines der parallelen Schenkel (17) des U-förmigen HF-Steckers (14) angeordnet ist und in mindestens einem der parallelen Schenkel (17) des U-förmigen HF-Steckers (14) eine Aufnahme (22) für die HF-Elektrode (6) ausgebildet ist.

2. Medizinisches HF-Instrument nach Anspruch 1, wobei die HF-Elektrode (6) nur bei in das Gehäuse (19) der Handhabe (5) eingedrücktem HF-Stecker (14) mit dem HF-Stecker (15) koppelbar ist.

3. Medizinisches HF-Instrument nach Anspruch 1 oder 2, wobei am mit dem HF-Stecker (14) koppelbaren Teil der HF-Elektrode (6) eine Rastfläche (26) ausgebildet ist, die im mit dem HF-Stecker (14) gekoppelten Zustand mit einer korrespondierenden Anlagefläche (27) im Gehäuse (19) der Handhabe (5) zusammenwirkt.

4. Medizinisches HF-Instrument nach Anspruch 1, wobei in beiden parallelen Schenkeln (17) des U-förmigen HF-Steckers (14) jeweils eine Aufnahme (22) für die HF-Elektrode (6) ausgebildet ist.

5. Medizinisches HF-Instrument nach Anspruch 1 wobei die mindestens eine Aufnahme (22) für die HF-Elektrode (6) distalseitig eine Anlaufschräge (30) sowie eine Dichtungslamelle (32) aufweist.

6. Medizinisches HF-Instrument nach Anspruch 1, wobei auf der Unterseite beider paralleler Schenkel (17) des U-förmigen HF-Steckers (6) jeweils mindestens zwei hintereinander angeordnete Federelemente (25) angeordnet sind.

## Claims

1. Medical HF instrument with a shaft (3), which has a hollow shaft tube (2), and with a handle (5) arranged at the proximal end (4) of the shaft tube (2), wherein an HF electrode (6) is mounted in the shaft tube (2) and, for the purpose of energy supply, can be coupled to an HF plug (14) that can be secured on the handle (5), wherein the HF electrode (6) is able to be secured on the HF plug (14) via a spring-loaded latching mechanism (24), wherein the spring-loaded latching mechanism (24) has at least one spring element (25) mounted on the HF plug (14), wherein the HF plug (14) can be pressed into a housing (19) of the handle (5) counter to the spring force of this at least one spring element (25), and wherein the HF plug (14) is U-shaped in cross section, wherein the at least one spring element (25) is arranged on an underside of at least one of the parallel limbs (17) of the U-shaped HF plug (14), said underside facing towards the handle (5), and a receptacle (22) for the HF electrode (6) is formed in at least one of the parallel limbs (17) of the U-shaped HF plug (14).

2. Medical HF instrument according to Claim 1, wherein the HF electrode (6) can be coupled to the HF plug (15) only when the HF plug (14) is pressed into the housing (19) of the handle (5).

3. Medical HF instrument according to Claim 1 or 2, wherein a latching surface (26) is formed on the part of the HF electrode (6) that can be coupled to the HF plug (14), which latching surface (26) interacts with a corresponding contact surface (27) in the housing (19) of the handle (5) in the state when coupled to the HF plug (14).

4. Medical HF instrument according to Claim 1, wherein a receptacle (22) for the HF electrode (6) is formed in each of the two parallel limbs (17) of the U-shaped HF plug (14).

5. Medical HF instrument according to Claim 1, wherein the at least one receptacle (22) for the HF electrode (6) has, at the distal end, a run-on bevel (30) and a sealing lamella (32).

6. Medical HF instrument according to Claim 1, wherein at least two spring elements (25) arranged one behind the other are arranged on the underside of both parallel limbs (17) of the U-shaped HF plug (6).

## Revendications

1. Instrument médical HF comprenant une tige (3) pourvue d'un tube de tige creux (2) et d'une poignée (5) disposée à l'extrémité proximale (4) du tube de tige (2), une électrode HF (6) étant montée dans le tube de tige (2) et pouvant être accouplée à un connecteur HF (14) qui peut être fixé à la poignée (5) pour l'alimentation en énergie, l'électrode HF (6) pouvant être fixée au connecteur HF (14) par le biais d'un mécanisme d'encliquetage à ressort (24), le mécanisme d'encliquetage à ressort (24) comportant au moins un élément à ressort (25) monté sur le connecteur HF (14), le connecteur HF (14) pouvant être enfoncé dans un boîtier (19) de la poignée (5) en s'opposant à la force de ressort de cet au moins un élément à ressort (25) et le connecteur HF (14) étant en forme de U en coupe transversale, l'au moins un élément à ressort (25) étant disposé sur un côté inférieur, dirigé vers la poignée (5), d'au moins une des branches parallèles (17) du connecteur HF (14) en forme de U et un logement (22) destiné à l'électrode HF (6) étant formé dans au moins une des branches parallèles (17) du connecteur HF (14) en forme de U.

2. Instrument médical HF selon la revendication 1, l'électrode HF (6) ne pouvant être accouplée au connecteur HF (15) que lorsque le connecteur HF (14) est enfoncé dans le boîtier (19) de la poignée (5).

3. Instrument médical HF selon la revendication 1 ou 2, une surface d'encliquetage (26) qui, à l'état accouplé au connecteur HF (14), coopère avec une surface d'appui correspondante (27) dans le boîtier (19) de la poignée (5), étant formée sur la partie de l'électrode HF (6) qui peut être accouplée au connecteur HF (14).

4. Instrument médical HF selon la revendication 1, un logement (22) destiné à l'électrode HF (6) étant formé dans chacune des deux branches parallèles (17) du connecteur HF (14) en forme de U.

5. Instrument médical HF selon la revendication 1, l'au moins un logement (22) destiné à l'électrode HF (6) comportant du côté distal un biseau d'engagement (30) et une lamelle d'étanchéité (32).

6. Instrument médical HF selon la revendication 1, au moins deux éléments à ressort (25) placés l'un derrière l'autre étant disposés sur le côté inférieur des deux branches parallèles (17) du connecteur HF (6) en forme de U.
